(19) Europäisches Patentamt
European Patent Office
Office européen des brevets

(11) **EP 1 923 447 A1**

(12) **EUROPEAN PATENT APPLICATION**
published in accordance with Art. 153(4) EPC

(43) Date of publication:
**21.05.2008 Bulletin 2008/21**

(51) Int Cl.:
**C09K 5/16** (2006.01)     **A61F 7/08** (2006.01)

(21) Application number: **06782487.0**

(22) Date of filing: **08.08.2006**

(86) International application number:
**PCT/JP2006/315661**

(87) International publication number:
**WO 2007/018211 (15.02.2007 Gazette 2007/07)**

(84) Designated Contracting States:
**DE FR GB**

(30) Priority: **09.08.2005 JP 2005231357**

(71) Applicant: **Kao Corporation**
**Chuo-ku**
**Tokyo 103-8210 (JP)**

(72) Inventors:
• **ISHIKAWA, Masataka**
  **Tochigi 321-3497 (JP)**

• **KUMAMOTO, Yoshiaki**
  **Tochigi 321-3497 (JP)**
• **OKA, Takeshi**
  **Tokyo 131-0044 (JP)**

(74) Representative: **HOFFMANN EITLE**
**Patent- und Rechtsanwälte**
**Arabellastrasse 4**
**81925 München (DE)**

(54) **HEATING ELEMENT AND HEATING INTERMEDIATE**

(57)     A heat generating element of the present invention contains an oxidizable metal, a moisture retaining agent, water, an electrolyte as an oxidation promoter, and a particulate hardening inhibitor. The particle size of the particulate hardening inhibitor is preferably 25% or smaller of that of the oxidizable metal. The heat generating element in which the oxidizable metal to the particulate hardening inhibitor mass ratio is preferably 1 to 30. The particulate hardening inhibitor preferably has a particle size of 10 μm or smaller.

EP 1 923 447 A1

**Description**

Technical Field

The present invention relates to a heat generating element utilizing heat generation accompanying oxidation reaction of an oxidizable metal with oxygen in air.

Background Art

A heat generating element making use of heat generation accompanying oxidation of iron powder, an oxidizable metal, with air oxygen has a problem that the iron powder cakes and hardens to lose flexibility with progress of the oxidation reaction. In particular, a heating element generating heat for several hours suffers appreciable reduction in flexibility because of sufficient progress of the oxidation of iron powder. In application to a body part of a user, the reduced flexibility not only gives discomfort to the user but also impairs efficient heat transfer to the body part.

Techniques that have been proposed to prevent such flexibility reduction include using zinc powder in place of iron powder thereby to maintain flexibility with progress of oxidation (see JP 2001-212167A, hereinafter "document 1") and combining a heat generating layer with a water-retaining gel layer to impart flexibility (see JP 2003-135509A, document 2).

It has also been proposed to incorporate a water soluble sodium silicate hydrate into a heat generating composition to prevent the composition from caking during use (see JP 3-47857B, document 3).

It has also been suggested to incorporate into a heat generating composition a powder of crystalline silicate aggregates having a specific surface area of 10 $m^2/g$ or more to prevent the composition from caking with the progress of metal powder (e.g., iron powder) oxidation (see JP 11-318966A, document 4).

The heat generating composition of document 1 does not contain a hardening inhibitor, and document 1 discloses no quantitative effects of hardening inhibition. The technique of document 2 involves provision of a water-retaining gel layer in addition to the heat generating layer, resulting in a so increased thickness. This not only reduces the comfort when the heat generating element is applied to a body part but also increases the production cost. The technique of document 3 is to make the heat generating composition strongly alkaline thereby hindering or retarding oxidation of metallic iron by the addition of a water soluble sodium silicate hydrate. That is, the addition affects the heat generating performance, resulting in, for example, a reduction of time period for maintaining an almost constant temperature. The crystalline silicate aggregate used in the technique of document 4 has a large surface unevenness by the very nature of being an aggregate and also contains voids between crystals. Therefore, oxidized iron powder is liable to adhere to the recesses or voids of the aggregates, making the silicate incapable of performing sufficient hardening inhibitory function.

Disclosure of the Invention

The present invention relates to a heat generating element that is designed to maintain flexibility to the end of the heat generation reaction without associated reduction in heat generating performance.

The present inventors have found that a particulate hardening inhibitor described later added to a heat generating element containing an oxidizable metal, a moisture retaining agent, water, and an electrolyte as an oxidation promoter inhibits hardening of the heat generating element accompanying progress of oxidation reaction without reducing the heat generating performance of the element. The present invention has been completed based on this finding.

Based on the above finding, the present invention provides a heat generating element containing an oxidizable metal, a moisture retaining agent, water, an electrolyte as an oxidation promoter, and a particulate hardening inhibitor the particle size of which is 25% or smaller of that of the oxidizable metal.

The invention also provides the heat generating element in which the oxidizable metal to hardening inhibitor mass ratio is 1 to 30.

The invention also provides a heat generating element precursor containing an oxidizable metal, a moisture retaining agent, and a particulate hardening inhibitor the particle size of which is 25% or smaller of that of the oxidizable metal. The precursor is free from an electrolyte as an oxidation promoter.

The invention also provides a heat generating element containing an oxidizable metal, a moisture retaining agent, water, an electrolyte as an oxidation promoter, and a hardening inhibitor and having a bending strength ratio of 6 or less. The bending strength ratio is a ratio of the bending strength at the end of heat generation reaction to the bending strength before heat generation reaction.

According to the present invention, a superior heat generating element that is designed to maintain flexibility up to the end of the heat generation reaction without associated reduction in heat generating performance and a precursor that is used to produce the heat generating element are provided.

Brief Description of the Drawing

Fig. 1 is a graph showing heat generation characteristics of the heat generating sheets obtained in Example 1 and Comparative Example 1.
Fig. 2 is a graph showing heat generation characteristics of the heat generating sheets obtained in Example 3 and Comparative Example 3.
Fig. 3 is a graph showing heat generation characteristics of the heat generating sheets obtained in Examples 5 and 6 and Comparative Example 1.
Fig. 4 is a graph showing heat generation characteristics of the heat generating sheets obtained in Example 9 and Comparative Example 6.

Detailed Description of the Invention

The present invention will be described based on its preferred embodiments with reference to the accompanying drawing.
The heat generating element according to the present embodiment contains an oxidizable metal, a moisture retaining agent, water, an electrolyte as an oxidization promoter, and a particulate hardening inhibitor. The heat generating element may further contain a fibrous material.
The oxidizable metal that can be used in the heat generating element may be any metal that generates heat on oxidation. Examples of such oxidizable metal include iron, aluminum, magnesium, copper, and zinc. Iron powder is preferred of them for easy control of exothermic reaction and low cost. Accordingly, the present invention will hereinafter be described based on an embodiment using iron powder as an oxidizable metal.
The iron powder that can be used in the present embodiment preferably has a particle size of 0.1 to 300 $\mu$m, more preferably 0.1 to 150 $\mu$m. As used herein, the term "particle size" denotes a maximum length of powder particles or an average particle size measured by a dynamic light scattering method, a laser diffraction method, and the like. The same applies to the particle size of the hardening inhibitor and moisture retaining agent. The iron powder particle size being within the recited range, oxidation reaction takes place efficiently.
In cases where the heat generating element contains a fibrous material and has the shape of a sheet as produced by a papermaking technique, it is preferred to use iron powder containing particles with a particle size of 0.1 to 300 $\mu$m, more preferably 0.1 to 150 $\mu$m, in a proportion of at least 50% by mass in view of good fixability on the fibrous material and reaction controllability.
The iron powder content in the heat generating element is preferably 10% to 95% by mass, more preferably 30% to 80% by mass. This range of iron power content brings about the following effects. The resulting heat generating element is capable of raising its temperature to a desired heat generation temperature, and, where the heat generating element is a heat generating sheet (described *infra),* the proportions of the fibrous material and a binding component (e.g., a flocculant) can be minimized to secure sufficient air permeability through the heat generating sheet. With sufficient air permeation, sufficient heat generation reaction propagates into the inside of the heat generating element to raise the heat generation temperature sufficiently high and achieve a sufficiently long duration of heat generation. Sufficient supply of moisture from the moisture retaining agent (hereinafter described) is secured. The iron powder hardly falls off. Since certain proportions of the fibrous material and binding component (hereinafter described) are secured, the resulting heat generating element has sufficient mechanical strength such as bending strength and tensile strength. The iron powder content in the heat generating element can be determined by ash content measurement in accordance with JIS P8128, thermogravimetry, or vibrating sample magnetization measurement (magnetization on applying an external magnetic field is made use of), and the like.
The content of the moisture retaining agent in the heat generating element is preferably 1% to 60% by mass, more preferably 3% to 50% by mass. This range of the moisture retaining agent content produces the following effects. A water content necessary to sustain the oxidation reaction can be stored in the heat generating element. Sufficient air permeation through the heat generating element is secured to provide sufficient oxygen supply and achieve high heat generation efficiency. The heat capacity of the heat generating element is suppressed with respect to the amount of heat generation, resulting in an enhanced, sufficient increase of temperature to a desired level. Where the heat generating element is a heat generating sheet described *infra,* the moisture retaining agent is prevented from falling off, and certain proportions of the fibrous material and binding component described *infra* are secured to provide sufficient mechanical strength, such as bending strength and tensile strength.
Any moisture retaining agents commonly employed in this type of heat generating elements can be used in the present invention with no particular limitation, including absorbent polymers and wood meal. Some moisture retaining agents not only serve for moisture retention but functions as an agent for holding and supplying oxygen to iron powder. Examples of such moisture retaining agents include activated carbon (palm shell charcoal, wood charcoal, bituminous coal, peat, and lignite), carbon black, acetylene black, graphite, zeolite, pearlite, vermiculite, silica, cancrinite, and fluorite.

Preferred of them is activated carbon for its moisture retaining ability, oxygen supplying ability, and catalytic ability. It is preferred to use a particulate moisture retaining agent having a particle size of 0.1 to 500 μm, particularly the one containing particles with a particle size of 0.1 to 200 μm in a proportion of at least 50% by mass, in view of the capability of providing an effective contact with iron powder. Moisture retaining agents of other forms are also usable. For example, those of fibrous form such as activated carbon fiber can be used.

The content of the electrolyte acting as an oxidation promoter in the heat generating element is preferably 0.5% to 30% by mass, more preferably 1% to 25% by mass, based on the water content of the resulting heat generating element. Within the recited range, the following effects are obtained. The oxidation reaction proceeds sufficiently in the resulting heat generating element. Precipitation of the electrolyte, which will impair air permeability through the heat generating element, hardly occurs. A necessary amount of the electrolyte needed for heat generation is secured. A sufficient amount of water is supplied to iron powder, etc. to assure high heat generating performance. The electrolyte can uniformly be distributed throughout the heat generating element.

The electrolyte to be added is not particularly limited, and any kind commonly used in this type of heat generating elements can be used. Examples of useful electrolytes include sulfates, carbonates, chlorides, and hydroxides of alkali metals, alkaline earth metals or heavy metals. Preferred of them are chlorides, such as sodium chloride, potassium chloride, calcium chloride, magnesium chloride, and iron (I) or (II) chloride, in view of their electrical conductivity, chemical stability, and production cost. These electrolytes can be used either individually or as a combination of two or more thereof.

In order to effectively prevent the heat generating element from hardening as a result of caking of iron powder due to oxidation, the particulate hardening inhibitor is preferably fine particles with a particle size in the range of from 0.01% to 25%, more preferably 0.02% to 20%, of the particle size of the iron powder.

The terminology "particulate" as used herein is intended to include such shapes as spherical, needle-like, platy, and columnar and exclude the shape of an aggregate as described in document 4 *supra.*

The particulate hardening inhibitor preferably has a particle size of 10 μm or smaller, more preferably 9 μm or smaller. It is particularly preferred to use the one containing at least 50% by mass of particles with a particle size of 0.1 to 10 μm, more preferably 0.5 to 9 μm. The smaller the particle size of the particulate hardening inhibitor, the more preferred. Nevertheless, the lower limit of the particle size is 0.1 μm, taking into consideration possible flying or scattering of components such as iron powder and possible fall-off of the components from a moisture permeable sheet if combined with the heat generating sheet as described later.

Examples of the particulate hardening inhibitor include carbon black, illite, cancrinite, talc, fluorite, bentonite, titanium oxide, and activated carbon having a specified particle size. Preferred of them are talc, fluorite, bentonite, and titanium oxide in view of their inhibitory effect on hardening of the heat generating element with the progress of oxidation without affecting the heat generation performance. Activated carbon, which doubles as a moisture retaining agent, is particularly preferred in terms of production cost.

When in using activated carbon having a specified particle size as a particulate hardening inhibitor, the heat generating element of the present invention contains an oxidizable metal, the moisture retaining agent of a specified particle size, water, and an electrolyte as an oxidation promoter. The activated carbon of a specified particle size is a constituent of the heat generating element which functions as both a moisture retaining agent and a particulate hardening inhibitor. Activated carbon as a moisture retaining agent and activated carbon as a hardening inhibitor may be used as blended. The constitution described above also applies to the heat generating element precursor.

In using activated carbon as a particulate hardening inhibitor, the mass ratio of the iron powder to the particulate hardening inhibitor is preferably 3 to 30, more preferably 5 to 20. With the ratio falling within the recited range, the inhibitory effect on hardening of the heat generating element due to oxidation-associated caking of iron powder is ensured, and the heat generating element can have a reduced thickness without largely affecting the heat generation performance. Where the heat generating element has a sheet form, not only the above described effects but an advantage that iron powder is well fixed to pulp fiber are obtained, which leads to higher sheet qualities, improved production stability, maintenance of mechanical strength, and reduced production cost. In using other particulate hardening inhibitors such as talc, the iron powder to hardening inhibitor mass ratio is preferably 1 to 30, more preferably 2 to 20, for the same reasons as mentioned.

The content of the particulate hardening inhibitor in the heat generating element is preferably 1% to 30% by mass, more preferably 3% to 50% by mass, when it is activated carbon or 3% to 50% by mass, more preferably 10% to 30% by mass, when it is talc.

It is desirable that the particulate hardening inhibitor be water insoluble for the following reason. When the heat generating element has a sheet form, the water insoluble hardening inhibitor can be added to a raw material composition that is used to make sheeting by a papermaking technique. When the heat generating element has a powder form (described latter), the water insoluble hardening inhibitor does not dissolve in existing water and keeps its powder form. Therefore, the hardening inhibitor continues physically preventing iron powder from caking onto neighboring components such as iron powder and the moisture retaining agent.

The water insolubility of the hardening inhibitor is preferably such that 0 to 1 g (by mass), more preferably 0 to 0.1 g, out of 10 g of the hardening inhibitor added to 100 g of water dissolves in the water.

It is desirable that the pH value of the particulate hardening inhibitor itself be out of the strongly alkaline region so that the hardening inhibitor may not hinder or delay oxidation of the oxidizable metal and thereby may not impair the heat generation performance of the heat generating element. Specifically, the particulate hardening inhibitor preferably has a pH of 3 to 10, more preferably 4 to 9. Within this pH range, the problem that heat generation is hindered, resulting in a failure to reach a desired temperature (e.g., 40°C or higher) can be eliminated. Furthermore, when the heat generating element is stored as packaged in an oxygen-impermeable bag, hydrogen evolution during storage and resultant burst of the bag can be avoided by that pH control.

The "pH value of the particulate hardening inhibitor itself" is a pH value of a mixture of 10 g (by mass) of the particulate hardening agent and 100 g of water.

In the case where the heat generating element is a heat generating sheet, the content of the fibrous material in the heat generating element is preferably 1% to 50% by mass, more preferably 3% to 40% by mass. Within this range, the other components including the iron powder, moisture retaining agent, and particulate hardening inhibitor are sufficiently prevented from falling off the sheet, and the sheet becomes suffice for a heat generating sheet. The heat capacity of the heat generating sheet is suppressed with respect to the amount of heat generation, resulting in an enhanced, sufficient increase of temperature. Moreover, certain proportions of the other components are secured in the heat generating element to obtain sufficient heat generation performance.

It is preferred for the fibrous material to have a CSF (Canadian Standard Freeness) of 600 ml or less, more preferably 450 ml or less. Fibrous materials having a CSF of 600 ml or less have sufficient fixing capabilities for the other components including iron powder, the moisture retaining agent, and the particulate hardening inhibitor to hold prescribed amounts of the components thereby to assure excellent heat generating performance. Furthermore, a sheet with a uniform thickness can be obtained. The sufficient fixing capabilities of the fibrous material for the other components will prevent fall-off of the components, and sufficient entanglement and hydrogen bonding between the fibrous material and the components will provide sufficient binding strength. As a result, the sheet has sufficient mechanical strength, such as bending strength and tensile strength, and excellent fabricability.

It is desirable for the fibrous material to have as low a CSF as possible. In carrying out ordinary papermaking using only pulp fiber, when the proportion of components other than the fibrous material is low, the CSF is preferably 100 ml or higher to secure satisfactory drainage and dewatering to provide a heat generating sheet with a uniform thickness. Moreover, molding defects such as burst of blisters on drying are hardly experienced. Since the proportion of the components other than the fibrous material is relatively high in the present invention, the composition shows satisfactory drainage to provide a heat generating sheet with a uniform thickness. A lower CSF indicates a higher fibril content, and a higher fibril content secures better fixation of the components other than the fibrous material on the fibrous material, which results in high sheet strength. The CSF of a fibrous material can be controlled by, for example, the degree of beating. The CSF may also be adjusted by blending fibers different in CSF.

CSF is measured by the method specified in JIS P8121 (pulps-determination of drainability). It is a measure of drainability of a fibrous material, taking a value of 0 or greater.

The fibrous material preferably has a negative zeta potential. "Zeta potential" is an apparent potential at the shear plane separating a charged particle and a solution, which can be determined by streaming potential measurement or electrophoresis measurement. A fibrous material having a negative zeta potential has satisfactory ability to fix the above-mentioned components including the iron metal, moisture retaining agent, and particulate hardening inhibitor, thereby to secure predetermined amounts of these components, assuring sufficient heat generating performance. Running of large quantities of the components into drain water can be prevented, which is favorable for the productivity and environmental protection.

The fibrous material preferably has an average length of 0.1 to 50 mm, more preferably 0.2 to 20 mm. When the average length is within that range, the following effects are obtained. The resulting heat generating element has sufficient mechanical strength, such as bending strength and tensile strength. The heat generating element is prevented from becoming so dense as to impair the air permeability. As a result, oxygen is adequately supplied to assure good heat generating performance. The fibrous materials are uniformly dispersed in the heat generating element to provide uniform mechanical strength and uniform sheet thickness. In addition, an appropriate interfiber distance is provided to hold the other components such as the iron powder, moisture retaining agent, and particulate hardening inhibitor, preventing these components from falling off.

Examples of the fibrous material include natural fibers, such as plant fibers (e.g., cotton, kapok fiber, wood pulp, non-wood pulp, peanut protein fiber, corn protein fiber, soybean protein fiber, mannan fiber, rubber fiber, hemp, Manila fiber, sisal fiber, New Zealand flax, Luo Buma, coconut, rush, and straw), animal fibers (e.g., wool, goat hair, mohair, cashmere, alpaca, angora, camel, vicuna, silk, down, small feather, alginate fiber, chitin fiber, and casein fiber), and mineral fibers (e.g., sepiolite, wollastonite, and rock wool); synthetic fibers, such as semi-synthetic fibers (e.g., cellulose diacetate, cellulose triacetate, oxidized cellulose acetate, promix, chlorinated rubber, and rubber hydrochloride); metal fibers; carbon fiber; and glass fiber. Also useful are single-component fibers made of polyolefin (e.g., high-density polyethylene, medium-density polyethylene, low-density polyethylene or polypropylene), polyester, polyvinylidene chloride, starch, polyvinyl alcohol or polyvinyl acetate, a copolymer thereof, or a modified product thereof; and sheath/core conjugate fibers having the above-recited resin component as a sheath. Of these fibers, polyolefin fibers and modified polyester fibers are preferably used for high bonding strength between individual fibers to form a three-dimensional network structure on fusion bonding, and their lower melting point than the ignition point of pulp fiber. Synthetic fibers of polymers having branches, such as branched polyolefin fibers, are also preferred for their fixing capabilities for the iron powder and moisture retaining agent. The above-recited fibrous materials can be used either individually or as a combination of two or more thereof. Recycled products of these fibrous materials are also employable. Among these fibrous materials particularly preferred are wood pulp and cotton in terms of their fixing capabilities for the iron powder and the moisture retaining agent, flexibility of the resulting heat generating element, oxygen permeability of the resulting heat generating element owing to the presence of interstitial voids, and the cost of production.

The water content of the heat generating element is preferably 5% to 80% (by mass, hereinafter the same), more preferably 10% to 60%. With the water content being in that range, a necessary amount of moisture needed for continuation of oxidation reaction is secured to prevent the oxidation reaction from coming to the end halfway. Furthermore, moisture can be distributed uniformly throughout the heat generating element to assure uniform heat generation. With a water content being 80% or less, the heat generating element has a controlled heat capacity for the amount of heat generated to achieve a sufficient rise in temperature, sufficient air permeability for achieving high heat generating performance, and sufficient shape retention and mechanical strength.

The raw material composition for making the heat generating element may contain other compositions in addition to the oxidizable metal, moisture retaining agent, water, electrolyte as an oxidation promoter, and particulate hardening inhibitor. For example, the raw material composition for making a heat generating sheet preferably contains a flocculant as described *infra*.

If desired, the heat generating element may contain additives commonly used in papermaking, such as sizes, colorants, strengthening agents, yield improvers, loading materials, thickeners, pH control agents, and bulking agents, with no particular limitation. The amounts of the additives to be added can be decided appropriately according to the kinds.

The process of producing a heat generating element will then be described with respect to its preferred embodiment in which a heat generating element of sheet form (i.e., a heat generating sheet) containing the above described fibrous material is produced.

Production of a heat generating sheet preferably starts with preparation of a raw material composition (slurry) containing the above described iron powder, moisture-retaining agent, particulate hardening inhibitor, fibrous material, and water, from which a precursor of a heat generating sheet (hereinafter "heat generating sheet precursor") is obtained by papermaking. Incorporating an electrolyte serving as an oxidation promoter into the heat generating sheet precursor provides a heat generating sheet as will be described later.

The raw material composition for making the heat generating sheet precursor is not limited to a composition containing the oxidizable powder, moisture retaining agent, and particulate hardening inhibitor and containing no electrolyte. It may contain other composition. For example, a flocculant is preferably added to the raw material composition as previously stated.

Flocculants which can be used in the present invention include inorganic ones, such as metal salts, e.g., ammonium sulfate, polyaluminum chloride, ferric chloride, polyferric sulfate, and ferrous sulfate; polymeric ones, such as polyacrylamides, sodium polyacrylates, Mannich base-modified polyacrylamide, aminoalkyl poly(meth)acrylates, sodium carboxymethyl celluloses, chitosans, starches, and polyamide-epichlorohydrins; organic flocculants, such as dimethyldiallylammonium chloride type or ethyleneimine type alkylene dichloride-polyalkylenepolyamine condensates, and dicyandiamide-formalin condensates; clay minerals, such as montmorillonite and bentonite; silicon dioxide and its hydrates, such as colloidal silica; and hydrous magnesium silicate, such as talc. Preferred of these flocculants are combinations of an anionic agent and a cationic agent in terms of sheet surface properties, formation, molding properties, powder (iron powder, moisture retaining agent, particulate hardening inhibitor, etc.) fixing properties, and sheet strength. Suitable combinations include a combination of colloidal silica or bentonite (anionic) and starch or polyacrylamide (cationic) and a combination of sodium carboxymethyl cellulose (anionic) and a polyamide-epichlorohydrin resin or polyacyrylamide (cationic). In addition to these combinations, the above-recited flocculants can be used either individually or in combination of two or more thereof.

As will be demonstrated in Example given later, bentonite recited above is also useful as a particulate hardening inhibitor as long as it has a particle size of 10 $\mu$m or smaller.

The flocculant is preferably used in an amount of 0.01% to 5% by mass, particularly 0.05 to 1% by mass, based on the total solids content of the raw material composition. At amounts of 0.01% by mass or more, the flocculant has good flocculating effect to prevent the components such as the iron powder, moisture retaining agent, and particulate hardening inhibitor from falling off during papermaking, give a uniform composition, and provide a sheet with a uniform thickness and composition. At amounts of 5% by mass or less, the flocculant's tendency to stick to drying rolls in the step of sheet drying and to cause breaking or scorching can be controlled to improve productivity. With 5% by mass or less of the flocculant, the raw material composition holds a good potential valence to minimize the loss of the above components into white water during papermaking. Moreover, oxidation may proceed in the sheet to provide good storage stability of heat generating performance and strength.

The raw material composition (slurry) concentration preferably ranges from 0.05% to 10% by mass, particularly 0.1% to 2% by mass. Within the recited preferred range, a large quantity of water is not required, much time is not needed for sheet forming, and a sheet with a uniform thickness can be formed. The slurry has a good disperse state to provide a heat generating sheet with excellent surface properties and a uniform thickness.

The slurry is made into a heat generating sheet precursor by a papermaking technique. Papermaking techniques include continuous papermaking by use of a cylinder paper machine, a foundrinier paper machine, a yankee paper machine, a twin-wire paper machine, etc.; and batch papermaking such as manual papermaking. A multi-layered, heat generating sheet precursor may be made by successively using slurries of different formulations or laminating sheets separately prepared from slurries of different formulations.

The heat generating sheet precursor as formed by papermaking is preferably dewatered to a water content of 70% or less (by mass, hereinafter the same), more preferably 60% or less, for assuring shape retention and mechanical strength. Dewatering is carried out by, for example, suction, application of pressurized air or pressing with a pressure roll or a pressure plate.

The dewatered sheet precursor, which contains the iron powder capable of exothermic reaction in an ordinary atmosphere, is then subjected to positive drying to remove the water content so as to inhibit oxidation of the iron powder during the subsequent fabrication steps and to provide a heat generating sheet precursor having excellent stability during long-term storage. Drying of the sheet is preferably carried out before addition of the aforementioned electrolyte (application of an electrolyte solution) so that the iron powder may be firmly fixed and held by the fibrous material and be prevented from falling off and that mechanical strength improvement by addition of a heat fusible component or a thermal crosslinking component may be expected.

The heat generating sheet precursor is preferably dried by heating. The heating temperature is preferably 60° to 300°C, more preferably 80° to 250°C. When the drying temperature is within this range, the following effects are obtained. The drying time is minimized, which is effective to suppress oxidation of the iron powder accompanying water removal. As a result, reduction of heat generating performance of the resulting heat generating sheet is prevented. Color change of the sheet due to oxidation of the iron powder is also prevented. Deterioration in performance of the moisture retaining agent, particulate hardening inhibitor, etc. can be suppressed, whereby heat generating performance of the heat generating sheet is maintained. Abrupt water vaporization inside the sheet, which can destroy the sheet structure, is avoided.

The water content of the heat generating sheet precursor after drying is preferably 20% or less, more preferably 10% or less. With the residual water content of 20% or less, the resulting sheet has good long-term storage stability. For example, when the sheet is temporarily stored in a roll form, water hardly migrates in the radial direction of the roll, causing no variations of heat generating performance and mechanical strength. It is preferred for the heat generating sheet precursor to have a breaking length of 100 to 4000 m, more preferably 200 to 3000 m. Within this range of breaking length, the sheet precursor is easy to handle during and after the production thereof, and the sheet precursor has moderate air permeability to significantly accelerate oxidation of the iron powder in the resulting heat generating element. The breaking length of a heat generating sheet precursor is measured as follows. A 15 mm wide and 150 mm long specimen cut out of a heat generating sheet precursor is subjected to a tensile test at an initial gauge length of 100 mm and a pulling speed of 20 mm/min in accordance with JIS P8113. A breaking length is calculated according to formula:

$$\text{Breaking length (m)} = (1/9.8) \times [(\text{tensile strength (N/m)}] \times 10^6/[\text{basis weight (g/m}^2)]$$

The heat generating sheet precursor free from an electrolyte as an oxidation promoter preferably has a basis weight of 10 to 1000 g/m$^2$, more preferably 50 to 600 g/m$^2$. A sheet basis weight in that range is light, comfortable to wear, and is stable during and after the production thereof.

The thickness of the heat generating sheet precursor is preferably 0.08 to 1.2 mm, more preferably 0.1 to 0.6 mm. The heat generating sheet precursor whose thickness falls within that range will exhibit good heat generation performance on addition of an electrolyte as an oxidation promoter and has sufficient mechanical strength, good fixation of the iron powder, moisture retaining agent, particulate hardening inhibitor, etc., and stable uniformity in thickness and compositional distribution. The sheet with that thickness hardly breaks due to pinhole development, showing good productivity and fabricability. The sheet has a good folding strength and hardly undergoes brittle fracture. The sheet has good flexibility to provide a good fit with no discomfort particularly when the resulting heat generating sheet is applied to a bending and stretching part of a body, such as elbows, knees, and the face. The sheet of the above thickness does not need a long time to form by papermaking and to dry, which is good for not only workability but heat generation performance and fabricability in, for example, bending.

A plurality of the heat generating sheet precursors thus prepared may be stacked one on top of another to provide a heat generating element that easily achieves heat generation performance as desired. The resulting heat generating element is thick and yet flexible and comfortable to use.

The method of drying the heat generating sheet precursor is selected appropriately depending on the sheet thickness, the treatment given to the sheet before drying, the water contents before and after the drying, and the like. Useful drying methods include contact with a heating unit, application of heated air or steam (superheated steam), vacuum drying, microwave heating, and electric current heating. The drying may be carried out simultaneously with the above-described dewatering.

The shaping (including dewatering and drying) of the heat generating sheet precursor is preferably conducted in an inert gas atmosphere. Containing no electrolyte as an oxidation promoter, the heat generating sheet precursor may also be shaped in an ordinary air atmosphere if desired, which enables simplification of equipment. Thin and yet tearproof, the resulting sheet precursor can be taken up in a roll where necessary.

If desired, the dried sheet precursor is fabricated by craping, slitting, trimming or perforating by needle punching. A thermoplastic resin component or a hot-water-soluble component may be incorporated into the slurry to facilitate heat sealing of the sheet precursor.

An electrolyte is then incorporated into the resulting heat generating sheet precursor. This step is preferably carried out in an inert gas atmosphere such as nitrogen or argon. Where the electrolyte is incorporated by impregnation with an electrolyte solution, the impregnating step may be conducted in an ordinary air atmosphere because oxidation that may proceed immediately after the impregnation is mild.

The method of incorporating an electrolyte into the heat generating sheet precursor is selected as appropriate to the treatment given to the sheet after sheet formation, the water content and form of the sheet, and the layer structure (in case of a multilayered structure) of the sheet, and so forth. For example, the electrolyte can be incorporated by impregnating the sheet precursor with a solution having a prescribed electrolyte concentration, or adding a powdered electrolyte having a prescribed particle size directly to the sheet precursor. Impregnation with an electrolyte solution having a prescribed concentration is preferred for achieving uniform distribution of the electrolyte and simultaneously controlling the water content of the resulting sheet.

When an electrolyte is incorporated into the sheet precursor by impregnation with a solution of the electrolyte, the manner of impregnation is chosen as appropriate to the form (e.g., thickness) and the water content of the sheet precursor. Impregnation methods include spraying, syringing into part of the sheet precursor (the injected electrolyte solution penetrates throughout the sheet by capillarity of the fibrous material), coating with a brush, etc., soaking in the electrolyte solution, gravure coating, reverse coating, doctor blade coating, and so on. Spraying is preferred for uniform distribution, ease of operation, and relatively low cost of equipment. Where the finished product has a complicated shape or layer structure, syringing is preferred for productivity, process flexibility (the final finishing process can be done in a separate step), and simplicity of equipment. It is possible to conduct syringing after the multilayered heat generating sheet is enclosed in a prescribed container.

After the electrolyte is incorporated, the water content of the sheet may be adjusted and stabilized according to need to provide a heat generating element (i.e., a heat generating sheet). If desired, the heat generating sheet thus prepared is sandwiched between a moisture permeable sheet and a moisture impermeable sheet, followed by trimming into a predetermined shape and size. The heat generating sheet may be sandwiched between a pair of moisture permeable sheets, followed by fabricating. The resulting heat generating element is supplied as packaged in, e.g., an oxygen-impermeable packaging material until use.

The single heat generating sheet preferably has a thickness of 0.08 to 2.0 mm, more preferably 0.15 to 1.8 mm. With a thickness of 0.08 mm or greater, the sheet has sufficient heat generating performance and mechanical strength. With a thickness of 2 mm or smaller, the sheet has sufficient flexibility and provides comfort to use. The thickness of the heat generating sheet is obtained by taking measurements at five or more different points in accordance with JIS P8118 and averaging the results.

The basis weight of the single heat generating sheet is preferably 10 to 2000 $g/m^2$, more preferably 50 to 1500 $g/m^2$. A sheet having basis weight of 10 $g/m^2$ or more can be formed with sufficient stability. A sheet having basis weight of 2000 $g/m^2$ or less is satisfactory in usability.

Two or more heat generating sheets may be used as stacked one on top of another. The thickness of the stack of the heat generating sheets is preferably 0.2 to 5 mm, more preferably 0.5 to 3 mm. A total thickness of 0.2 mm or larger secures sufficient heat generating performance and mechanical strength. A total thickness of 5 mm or smaller secures sufficient flexibility and usability.

The stack of the heat generating sheets preferably has a basis weight of 100 to 3000 $g/m^2$, more preferably 200 to 2500 $g/m^2$. With basis weight of the stack falling within this range, the stack exhibit excellent heat generating performance and initial usability.

It is preferred for the stack of the heat generating sheets to have a density of 0.6 to 3 g/cm$^3$, more preferably 0.7 to 2 g/cm$^3$. With a density of 0.6 g/cm$^3$ or higher, the oxidizable metal and other components are in sufficiently close contact with each other, which is favorable for obtaining good heat generating performance and sufficient strength, and the constituent components hardly fall off, which is favorable in terms of productivity and fabricability. With a density of 3 g/cm$^3$ or lower, the stack has satisfactory flexibility and comfort to use, and hardening with the progress of oxidation is alleviated.

Since the heat generating element of the present embodiment contains the particulate hardening inhibitor, it remains flexible even with the progress of oxidation and has satisfactory heat generating performance. The effect of the hardening inhibitor in the heat generating element is evaluated in terms of bending strength ratio obtained in the bending strength measurements described later. The bending strength ratio is preferably 1 to 6, more preferably 2 to 6. When the bending strength ratio is in this range, the heat generating element retains flexibility and good fit against an object to which it is applied, e.g., a body part, up to the end of its heat generation reaction. When applied to a body part, in particular, the heat generating element efficiently transfers its heat to the body part without making the wearer feel uncomfortable.

The present invention is not construed as being limited to the aforementioned embodiment, and various modifications can be made thereto without departing from the spirit and scope of the invention. The invention is applicable to not only a heat generating sheet but a commercially widely available disposable body warmer that uses a heat generating element of powder form (a powdered heat generating element containing iron powder, a moisture retaining agent, etc. put in a bag made of, e.g., a moisture permeable film), a thermotherapeutic pad, and the like. In the case of a heat generating element of powder form, too, a precursor free from an electrolyte as an oxidation promoter may be prepared first, which is then provided with an electrolyte to give a heat generating element.

Similarly to the stack of the heat generating sheets, the heat generating element of powder form preferably has a basis weight of 100 to 3000 g/m$^2$, more preferably 200 to 2500 g/m$^2$. With the basis weight of the heat generating element of powder form falling within this range, the element exhibits excellent heat generating performance and initial usability.

Similarly to the stack of the heat generating sheets, the heat generating element of powder form preferably has a density of 0.6 to 3 g/cm$^3$, more preferably 0.7 to 2 g/cm$^3$. With the density between 0.6 g/cm$^3$ and 3 g/cm$^3$, the heat generating element of powder form has good flexibility, and hardening with the progress of oxidation is alleviated.

Examples

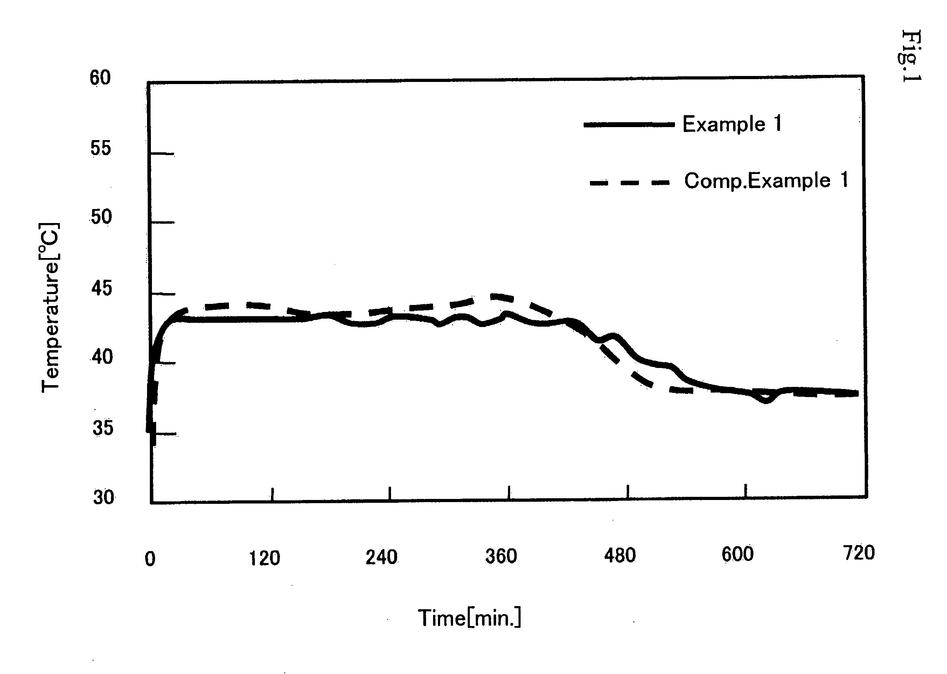

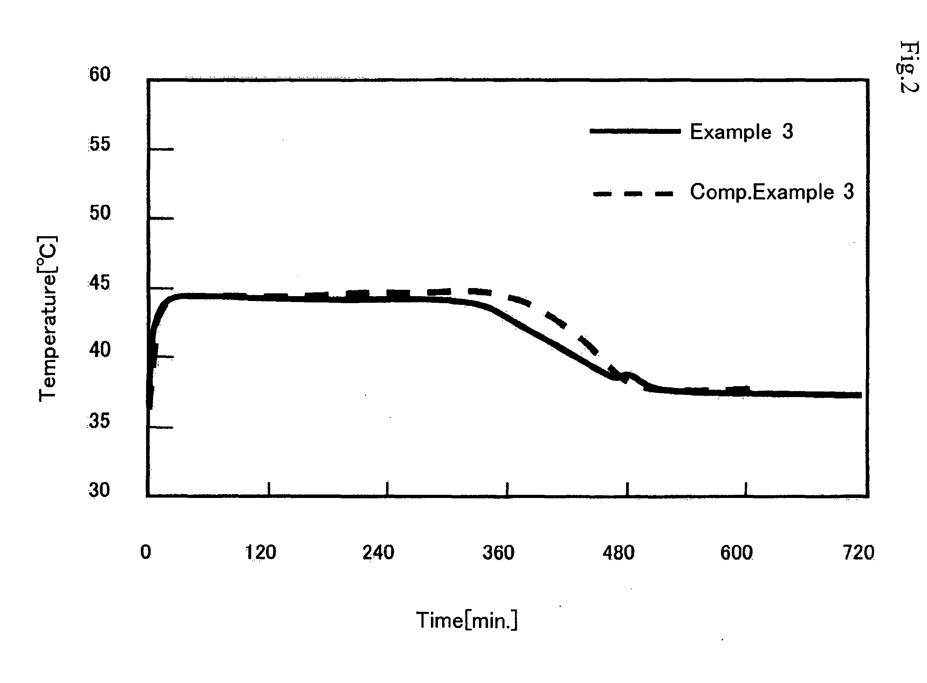

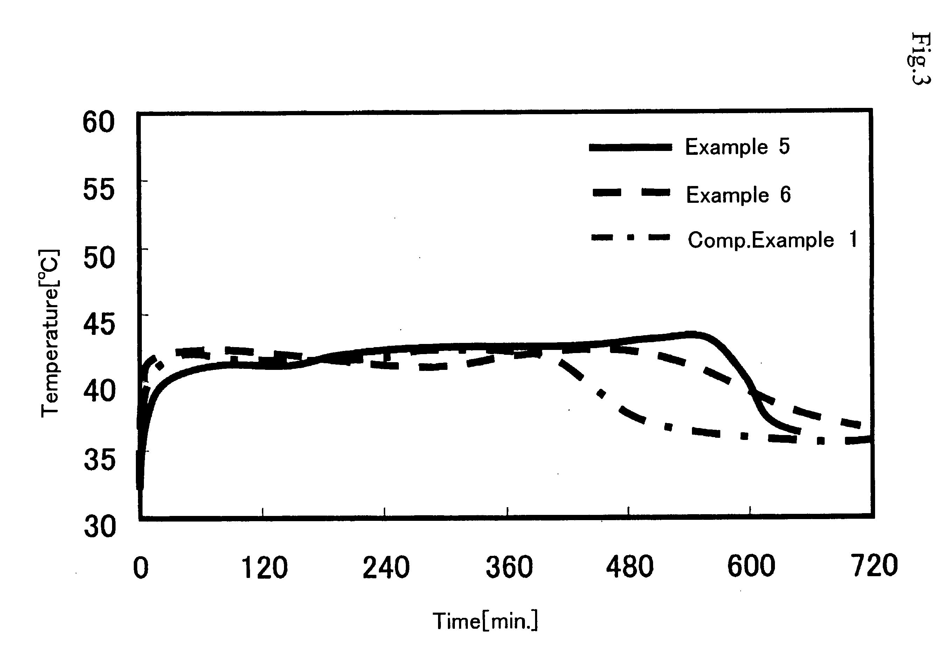

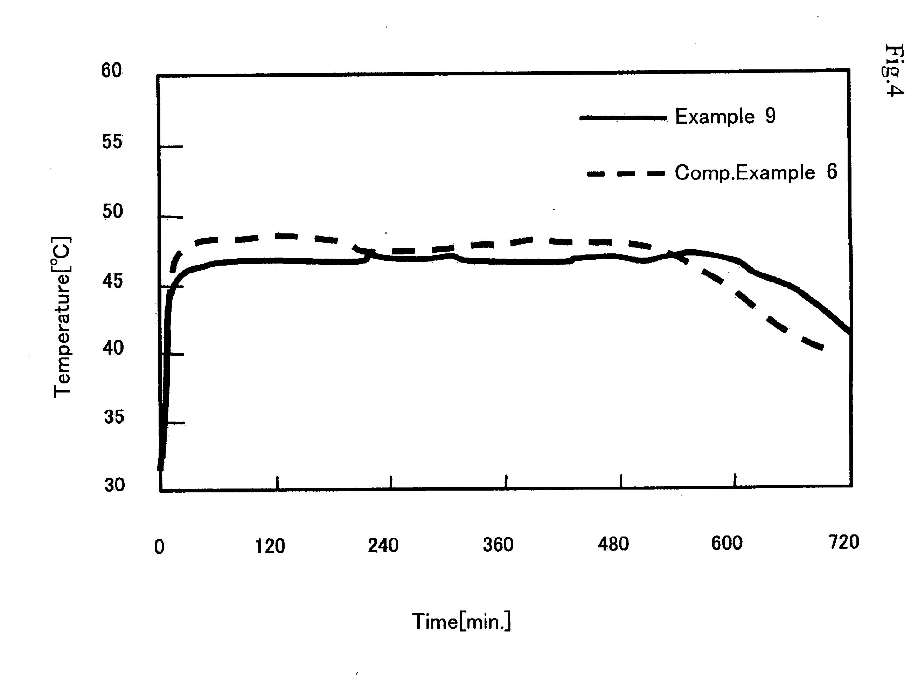

The heat generating element of the invention will now be illustrated in greater detail with reference to Examples. Examples 1 to 6 and Comparative Examples 1 to 4 show examples of the heat generating element of sheet form, and Examples 7 to 9 and Comparative Examples 5 and 6 illustrate examples of heat generating element of powder form.
Heat generating sheet precursors the solids compositions of which are shown in Table 1 below were prepared according to the procedures described in Examples 1 to 6 and Comparative Examples 1 to 4. Heat generating sheets were obtained from the respective sheet precursors as described below.
Heat generating elements of powder form were produced as described in Examples 7 to 9 and Comparative Examples 5 and 6 using a commercially available heat generating powder composition or a prepared heat generating powder composition, the formulations of which are shown in Table 3 below.
The resulting heat generating elements were evaluated for flexibility by measuring bending strength (maximum bending load) before and after oxidation (heat generating reaction) as described below. The results obtained are shown in Tables 2 and 4. The heat generating elements were also evaluated for heat generation characteristics as described below. The results are shown in Figs. 1 through 4.

Example 1

(1) Formulation of raw material composition

| | |
|---|---|
| Iron powder RKH (trade name) from Dowa Iron Powder Co., Ltd., particle size: 45 $\mu$m | 25.2 g |
| Fibrous material: pulp fiber Mackenzie (trade name) from Fletcher Challenge Canada, Ltd. | 2.4 g |
| Moisture retaining agent: activated carbon Carboraffin (trade name) from Japan EnviroChemicals, Ltd.; particle size: 45 $\mu$m | 2.4 g |
| Particulate hardening inhibitor: talc SG2000 (trade name) from Nippon Talc Co., Ltd.; particle size: 0.9 $\mu$m | 1.5 g |

The raw material composition was agitated at 300 rpm for 1 minute and drained through a square type standard sheet machine fitted with an 80 mesh wire manufactured by Kumagai Riki Kogyo K.K. in accordance with JIS P8209. The resulting wet sheet was dried on a KRK rotary drier (from Kumagai Riki Kogyo) to a water content of 1% by mass or lower to obtain a heat generating sheet precursor. The basis weight of the resulting sheet was about 450 g/m$^2$.

(2) Preparation of heat generating sheet

The resulting heat generating sheet precursor was cut into 80 mm by 100 mm rectangles. Two rectangular sheets were stacked on each other, and the electrolyte solution was syringed into the stack to penetrate throughout the stack by capillarity to obtain a heat generating sheet stack containing 37.5% of the electrolyte solution based on the mass of the heat sheet precursor stack. A moisture permeable sheet (microporous polyethylene sheet having a water vapor transmission rate of 1000 g/(m$^2$·24 hr) and a moisture impermeable sheet (polyethylene sheet) were superposed on one side and the other side, respectively, of the stack and joined together around the perimeter of the stack to obtain a test sample.

(3) Evaluation on heat generating reaction

The resulting test sample was allowed to generate heat, and the heat generation temperature was measured with a simplified temperature measuring device according to JIS S4100. The simplified temperature measuring device is a horizontal measuring stage having stacked thereon six polypropylene sheets each having a thickness of 1 mm and two thicknesses of gauze (Japan Pharmacopoeia type I) in that order and having its surface maintained at 35°C. The test sample was placed on the device with the moisture permeable sheet down, and eight thicknesses of 100% cotton flannel (5.905 tex, double yarn) were overlaid thereon, and the heat generation temperature was measured.
As a result, heat generation at 40°C or higher temperature lasted for 5 hours or longer. Then, the temperature of the heat generating sheet began to drop. The time point when the temperature dropped to 40°C was taken as the end point of the heat generating reaction. By the end of the heat generating reaction, the heat generating sheet had turned reddish brown all over, indicating overall progress of oxidation.

(4) Measurement of bending strength

The test sample, before and after the heat generating reaction, was supported on both of their ends at a span length of 50 mm and pressed at the middle with an indenter having a width of 50 mm and a tip radius of 5 mm at a crosshead speed of 20 mm/min. The maximum bending load was recorded as a bending strength. A bending strength ratio (bending strength at the end of heat generation reaction to the bending strength before heat generation reaction) was calculated.

Example 2

A heat generating sheet was obtained in the same manner as in Example 1, except for increasing the talc to 30 parts by mass as shown in Table 1.

Examples 3 and 4

A heat generating sheet was obtained in the same manner as in Example 1, except for replacing the moisture retaining agent and particulate hardening inhibitor used in Example 1 with the activated carbon described below, changing the formulation as shown in Table 1, and changing the amount of the electrolyte solution as shown in Table 2.
Moisture retaining agent/particulate hardening inhibitor: activated carbon Taiko SA1000 (trade name) available from Futamura Chemical Industries, Co., Ltd.; particle size: 9 μm; amount: 3.3 g (Example 3) or 2.4 g (Example 4).

Example 5

A heat generating sheet was obtained in the same manner as in Example 1, except for replacing talc with bentonite and changing the formulation as shown in Table 1.
Particulate hardening inhibitor: bentonite Bengel W-200U (trade name) available from Hojun Co., Ltd.; average particle size: 2 μm or smaller

Example 6

A heat generating sheet was obtained in the same manner as in Example 1, except for replacing talc with titanium oxide and changing the formulation as shown in Table 1.
Particulate hardening inhibitor: titanium oxide SANKA TITAN(IV) Anatase type (trade name) available from Kanto Chemical Co., Inc.; particle size: 2 μm

Comparative Example 1

A heat generating sheet was prepared in the same manner as in Example 1, except for using no talc. The flexibility at the end of heat generation reaction was examined.

Comparative Example 2

A heat generating sheet was prepared in the same manner as in Example 1, except for using talc having a particle size of 13 μm. The flexibility at the end of heat generation reaction was examined.

Comparative Examples 3 and 4

A heat generating sheet was prepared in the same manner as in Example 1, except for changing the compounding ratios of the moisture retaining agent and pulp as shown in Table 1.

Example 7

(1) Formulation of raw material composition

| | |
|---|---|
| Iron powder RKH (trade name) from Dowa Iron Powder Co., Ltd., particle size: 45 μm | 8 g |
| Moisture retaining agent: vermiculite from Chiyoda Cera Co., Ltd. (product class: 0); maximum particle size: <500 μm (catalogue value)) | 1 g |
| Particulate hardening inhibitor: activated carbon Taiko SA1000 (trade name) from Futamura Chemical Industries, Co., Ltd.; particle size: 9 μm) | 1 g |
| 5% NaCl solution | 5 g |

(2) Preparation of heat generating element

The above raw materials were thoroughly mixed in a nitrogen atmosphere, and the mixture was evenly packed into an 80 mm by 100 mm bag made by superposing the same moisture permeable sheet and moisture impermeable sheet as used in Example 1.
The resulting heat generating element of powder form was evaluated in the same manner as in Example 1.
In measuring bending strength of a heat generating element of powder form, cases are sometimes met with in which the applied load of the indenter once drops as a result of a break of the element and then again increases. In such cases, the load at the break is taken as a bending strength instead of the maximum load.

Example 8

Formulation of raw material composition

| | |
|---|---|
| Iron powder RKH (trade name) from Dowa Iron Powder Co., Ltd.; particle size: 45 μm | 8 g |
| Moisture retaining agent A: activated carbon Carboraffin (trade name) from Japan EnviroChemicals, Ltd.; particle size: 45 μm | 1 g |
| Moisture retaining agent B: vermiculite from Chiyoda Cera Co., Ltd.; product class: 0; particle size: <500 μm | 1 g |
| Particulate hardening inhibitor: talc SG2000 (trade name) from Nippon Talc Co., Ltd.; particle size: 0.9 μm | 1 g |

(continued)

| 5% NaCl solution | 5 g |

A heat generating element was prepared using the above raw materials and evaluated in the same manner as in Example 7.

Example 9

The same talc as used in Example 1 was mixed into the heat generating composition of a commercially available heat generating element of powder form (Jikabari (trade name) from Hisamitsu Pharmaceutical Co., Inc.) in an amount of 3.6 g per 18 g of solids content of 23 g of the heat generating composition. A heat generating element was prepared in the same manner as in Example 1, except for using the resulting mixed powder and putting the mixed powder into the same bag as used in the commercial product Jikabari. The moisture permeable side of the bag had a water vapor transmission rate of about 200 to 300 g/(m$^2$·24 hr). The resulting heat generating element was allowed to generate heat with its moisture permeable side up. This is the way adopted in using an ordinary heat generating element of powder form.

Comparative Example 5

A heat generating element was prepared in the same manner as in Example 8, except that the particulate hardening inhibitor was not added.

Comparative Example 6

A heat generating element was prepared in the same manner as in Example 5, except for using 23 g of the heat generating composition of a commercially available heat generating element of powder form (Jikabari from Hisamitsu).
Table 1

| | Solids Composition of Heat Generating Sheet Precursor (mass%) | | | External Additive | | Iron Powder Particle Size (μm) | Moisture Retaining Agent Particle Size (μm) | Hardening Inhibitor Particle Size (μm) | Iron Powder/ Hardening Inhibitor Mass Ratio |
|---|---|---|---|---|---|---|---|---|---|
| | Iron Powder | Moisture Retaining Agent | Pulp | Kind | Amount (part by mass*1) | | | | |
| Example 1 | 84 | 8 | 8 | talc | 5 | 45 | 45 | 0.9 | 16.8 |
| Example 2 | 84 | 8 | 8 | talc | 30 | 45 | 45 | 0.9 | 2.8 |
| Example 3 | 84 | 11 | 5 | - | - | 45 | 9 | | 7.6 |
| Example 4 | 84 | 8 | 8 | - | - | 45 | 9 | | 10.5 |
| Example 5 | 84 | 8 | 8 | bentonite | 10 | 45 | 45 | 2 or less | 8.4 |
| Example 6 | 84 | 8 | 8 | titanium oxide | 10 | 45 | 45 | 2 or less | 8.4 |
| Compara. Example 1 | 84 | 8 | 8 | - | - | 45 | 45 | - | - |
| Compara. Example 2 | 84 | 8 | 8 | talc | 5 | 45 | 45 | 13 | 16.8 |
| Compara. Example 3 | 84 | 11 | 5 | - | - | 45 | 45 | - | - |
| Compara. Example 4 | 84 | 5 | 11 | - | - | 45 | 45 | - | - |
| Note *1: Per 100 parts of the main constituent components of the precursor. | | | | | | | | | |

Table 2

| | Electrolyte Solution (5% NaCl, externally added) (part by mass*2) | Total Thickness of Heat Generating Sheet (mm) | Bending Strength (N/8 cm) | | Bending Strength Ratio |
|---|---|---|---|---|---|
| | | | Before Heat Generating Reaction | After Heat Generating Reaction | |
| Example 1 | 60 | 1.2 | 0.5 | 2.6 | 5.2 |
| Example 2 | 60 | 1.5 | 0.5 | 1.9 | 3.8 |
| Example 3 | 50 | 1.0 | 0.5 | 2.0 | 4.0 |
| Example 4 | 50 | 1.0 | 0.4 | 2.1 | 5.3 |
| Example 5 | 50 | 1.3 | 0.5 | 2.4 | 4.8 |
| Example 6 | 50 | 1.0 | 0.5 | 2.3 | 4.6 |
| Compara. Example 1 | 60 | 1.0 | 0.3 | 3.5 | 11.7 |
| Compara. Example 2 | 60 | 1.1 | 0.4 | 4.4 | 11.0 |
| Compara. Example 3 | 50 | 1.1 | 0.4 | 4.6 | 11.5 |
| Compara. Example 4 | 50 | 1.0 | 0.3 | 5.4 | 18.0 |
| Note *2: Per 100 parts of the sum of the main constituent components of the precursor and the external additive | | | | | |

Table 3

| | Solids Composition of Heat Generating Element (mass%) | | | External Additive | | Iron Powder Particle Size (μm) | Moisture Retaining AgentParticle Size (μm) | Hardening Inhibitor Particle Size (μm) | Iron Powder/ Hardening Inhibitor Mass Ratio |
|---|---|---|---|---|---|---|---|---|---|
| | Iron Powder | Moisture Retaining Agent | Pulp | Kind | Amount (part by mass*4) | | | | |
| Example 7 | 89 | 11 | - | activated carbon | 11 | 45 | <500*6 | 9 | 8.1 |
| Example 8 | 80 | 20 | - | Talc | 10 | 45 | *5 | 0.9 | 8.0 |
| Example 9 | *3 | *3 | - | Talc | 20 | *3 | *3 | 0.9 | *3 |
| Compara. Example 5 | 80 | 20 | - | - | - | 45 | 45 | - | - |
| Compara. Example 6 | *3 | *3 | - | - | - | *3 | *3 | - | - |

Note:
*3: The heat generating powder of a commercially available (Jikabari from Hisamitsu) was used as such.
*4: Per 100 parts of the main constituent components of the heat generating element.
*5: Moisture retaining agent A=activated carbon; particle size: 45 μm; moisture retaining agent B=vermiculite; particle size: 500 μm or smaller.
*6: The maximum value according to the catalogue.

Table 4

| | Electrolyte Solution (5% NaCl, externally added) (part by mass*7) | Thickness of Heat Generating Element (mm) | Bending Strength (N/8 cm) | | Bending Strength Ratio |
| --- | --- | --- | --- | --- | --- |
| | | | Before Heat Generating Reaction | After Heat Generating Reaction | |
| Example 7 | 56 | 1.9 | 0.6 | 3.1 | 5.3 |
| Example 8 | 50 | 2.0 | 0.6 | 3.2 | 5.4 |
| Example 9 | *3 | 2.7 | 2.4 | 6.6 | 2.8 |
| Compara. Example 5 | 50 | 2.0 | 0.4 | 3.4 | 8.8 |
| Compara. Example 6 | *3 | 2.7 | 2.0 | 13.1 | 6.6 |

Note
*3: The heat generating powder of a commercially available (Jikabari from Hisamitsu) was used as such.
*7: Per 100 parts of the sum of the main constituent components of the heat generating element and the external additive.

The results in Tables 2 and 4 provide confirmation that the heat generating elements of Examples retain flexibility up to the end of the heat generating reaction as compared with those of Comparative Examples even with their total thickness being practically equal. It is also confirmed that the heat generating elements of Examples have a bending strength ratio of 6 or smaller, indicating a higher flexibility retention than those of Comparative Examples. As graphically shown in Figs. 1 through 4, the heat generating elements of Examples prove equal in heat generation performance to those of Comparative Examples. In other words, the present invention provides a superior heat generating element that remains flexible even at the end of the heat generating reaction without involving reduction of heat generation performance.

Industrial Applicability

The heat generating element according to the present invention is thin, flexible before and after use, and heats up in a short time. Taking advantage of these characteristics, the heat generating element of the invention can find wide applications as such or as combined with various functional preparations. For example, it is combined with preparations for facial or body cleaning, sterilization or moisturization, slow wax release, scenting or deodorization to provide articles for skin care applications such as towelettes, steam generating articles, facial packs, make-up removing wipes; articles for home care applications for cleaning or treatment of flooring, *tatami,* interior goods, kitchen equipment (e.g., stoves and fans); articles for car care applications for cleaning and waxing; and the like. The heat generating element of the invention may be designed to have a moisture permeable side to serve as a steam generating article that supplies steam from its moisture permeable side to a desired object. The steam generated improves the warming effect and cleaning effect of the heat generating element.

**Claims**

1. A heat generating element comprising an oxidizable metal, a moisture retaining agent, water, an electrolyte as an oxidation promoter, and a particulate hardening inhibitor, the particulate hardening inhibitor having a particle size of 25% or smaller of that of the oxidizable metal.

2. The heat generating element according to claim 1, wherein the oxidizable metal is iron powder.

3. The heat generating element according to claim 1, wherein the oxidizable metal to the hardening inhibitor mass ratio is 1 to 30.

**4.** The heat generating element according to any one of claims 1 to 3, wherein the particulate hardening inhibitor has a particle size of 10 $\mu$m or smaller.

**5.** The heat generating element according to any one of claims 1 to 4, wherein the particulate hardening inhibitor is activated carbon.

**6.** A heat generating element precursor comprising an oxidizable metal, a moisture retaining agent, and a particulate hardening inhibitor and being free from an electrolyte as an oxidation promoter, the particulate hardening inhibitor having a particle size of 25% or smaller of that of the oxidizable metal.

**7.** The heat generating element according to claim 6, wherein the oxidizable metal is iron powder.

**8.** The heat generating element according to claim 6, wherein the oxidizable metal to hardening inhibitor mass ratio is 1 to 30.

**9.** The heat generating element according to any one of claims 6 to 8, wherein the particulate hardening inhibitor has a particle size of 10 $\mu$m or smaller.

**10.** The heat generating element according to any one of claims 6 to 9, wherein the particulate hardening inhibitor is activated carbon.

**11.** A heat generating element comprising an oxidizable metal, a moisture retaining agent, water, an electrolyte as an oxidation promoter, and a hardening inhibitor and having a bending strength ratio of 6 or less, the bending strength ratio being a ratio of bending strength at the end of heat generation reaction to bending strength before heat generation reaction.

Fig.1

Fig.2

Fig.3

Fig.4

**INTERNATIONAL SEARCH REPORT**

| International application No. |
| --- |
| PCT/JP2006/315661 |

A. CLASSIFICATION OF SUBJECT MATTER
*C09K5/16(2006.01)i, A61F7/08(2006.01)i*

According to International Patent Classification (IPC) or to both national classification and IPC

B. FIELDS SEARCHED

Minimum documentation searched (classification system followed by classification symbols)
C09K5/16, A61F7/08

Documentation searched other than minimum documentation to the extent that such documents are included in the fields searched
Jitsuyo Shinan Koho       1922-1996   Jitsuyo Shinan Toroku Koho   1996-2006
Kokai Jitsuyo Shinan Koho   1971-2006   Toroku Jitsuyo Shinan Koho   1994-2006

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| X | JP 2004-143232 A  (Kao Corp.),<br>20 May, 2004 (20.05.04),<br>Example 1<br>(Family: none) | 1-11 |
| X | JP 11-318966 A  (Kao Corp.),<br>24 November, 1999 (24.11.99),<br>Examples 1 to 4; Fig. 4<br>(Family: none) | 1-5,11 |
| P,X | JP 2005-224312 A  (Kao Corp.),<br>25 August, 2005 (25.08.05),<br>Claim 1; Par. No. [0011]; examples 1 to 2<br>(Family: none) | 1-11 |

☐  Further documents are listed in the continuation of Box C.      ☐  See patent family annex.

| * | Special categories of cited documents: |
| --- | --- |
| "A" | document defining the general state of the art which is not considered   to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>    31 October, 2006 (31.10.06) | Date of mailing of the international search report<br>    07 November, 2006 (07.11.06) |
| --- | --- |
| Name and mailing address of the ISA/<br>    Japanese Patent Office | Authorized officer |
| Facsimile No. | Telephone No. |

Form PCT/ISA/210 (second sheet) (April 2005)

**EP 1 923 447 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001212167 A
- JP 2003135509 A
- JP 3047857 B
- JP 11318966 A